# EUROPÄISCHE PATENTSCHRIFT

(11) **EP 0 548 833 B1**
(45) Veröffentlichungstag und Bekanntmachung des Hinweises auf die Patenterteilung: **11.09.1996**
(21) Anmeldenummer: 92121572.9
(22) Anmeldetag: 18.12.1992
(51) Int. Cl.: A61B 5/11

(54) **Medizintechnisches Gerät zur Diagnose und Therapie von Schlafstörungen**
Medical device for diagnostics and treatment of sleep disorders
Dispositif médical destiné au diagnostic et à la thérapie de l'insomnie

(30) Priorität: 20.12.1991 DE 4142427
(43) Veröffentlichungstag der Anmeldung: 30.06.1993
(73) Patentinhaber: Dokoupil, Hans, Dipl.-Ing. Univ., D-73342 Bad Ditzenbach (DE)
(72) Erfinder: Dokoupil, Hans, Dipl.-Ing. Univ., D-73342 Bad Ditzenbach (DE)

(56) Entgegenhaltungen:
- EP-A- 0 244 933
- WO-A-92/19318
- GB-A- 427 037
- MEDICAL & BIOLOGICAL ENGINEERING Bd. 17, Nr. 3, Mai 1979, STEVENAGE (GB) Seiten 416 - 418 A. HIGASHI ET AL. 'Real time online data processing system for the e.e.g.and..'

## Beschreibung

Eine genaue Diagnose von Schlafstörungen wird klinisch in Schlaflabors erstellt, indem vom Patienten ein Elektroencephalogramm (EEG) abgeleitet wird, das der Arzt auswertet.

Zwei Geräte (der Firma Madaus Medizin-Elektronik, 7803 Gundelfingen, sowie der Firma Medanz; Zeitschrift "Medical Tribune", Jahrgang 1991, Heft Nr. 29, Seite 14) mit denen man nur eine spezielle Schlafstörung, das Schlafapnoe-Syndrom ambulant diegnostizieren kann, registrieren die Herzfrequenz, die Schnarchgeräusche, die Schlafposition und die Sauerstoff-Sättigung, sowie über eine Gesichtsmaske den nasalen und oralen Luftstrom.

Die zwei oben erwähnten Geräte können jedoch nicht vom Patienten selbst bedient werden, da die verschiedenen Elektroden bzw. Sensoren vom medizinischen Fachpersonal angelegt werden müssen.

Der Erfindung liegt das Problem zugrunde, ein kostengünstiges, im häuslichen Bereich einsetzbares Gerät zur Diagnose und Therapie von Schlafstörungen zu schaffen, das vom Patienten in einfacher Weise selbst bedient werden kann.

Die Aufgabe wird durch ein im Anspruch 1 gekennzeichnetes Gerät gelöst.

Die mit der Erfindung erzielten Vorteile bestehen insbesondere darin, daß der Patient das Gerät selbst bedienen kann, indem der Wachzustand durch Betätigen eines Druckschalters registriert wird und dadurch keine Elektroden oder Sensoren vom medizinischen Fachpersonal angelegt werden müssen. Die notwendige Muskelan- und entspannung zum Betätigen des Druckschalters ergibt hier zusätzlich die Jacobson-Therapie (Jacobson, E., Progressive Relaxation, University of Chicago Press, 1928).

Eine vorteilhafte Ausgestaltung der Erfindung ist im Anspruch 2 angegeben. Die Weiterbildung nach Anspruch 2 ermöglicht durch den Gummischlauch (4) eine maximale elektrische Sicherheit für den Patienten.

Eine weitere vorteilhafte Ausgestaltung der Erfindung ist im Anspruch 3 angegeben. Die Weiterbildung nach Anspruch 3 ermöglicht einen guten Kompromis zwischen großer Zeitauflösung und Einsparung von Speicherkapazität.

Eine weitere vorteilhafte Ausgestaltung der Erfindung ist im Anspruch 4 angegeben. Die Weiterbildung nach Anspruch 4 ermöglicht es, daß die gespeicherten Daten nur durch den Arzt ausgedruckt werden können. Außerdem ergibt sich eine Kosteneinsparung, da ein Arzt gewöhnlich mehrere Patienten mit Registriergeräten (1) betreut, aber nur ein Ausdruckgerät (2) benötigt.

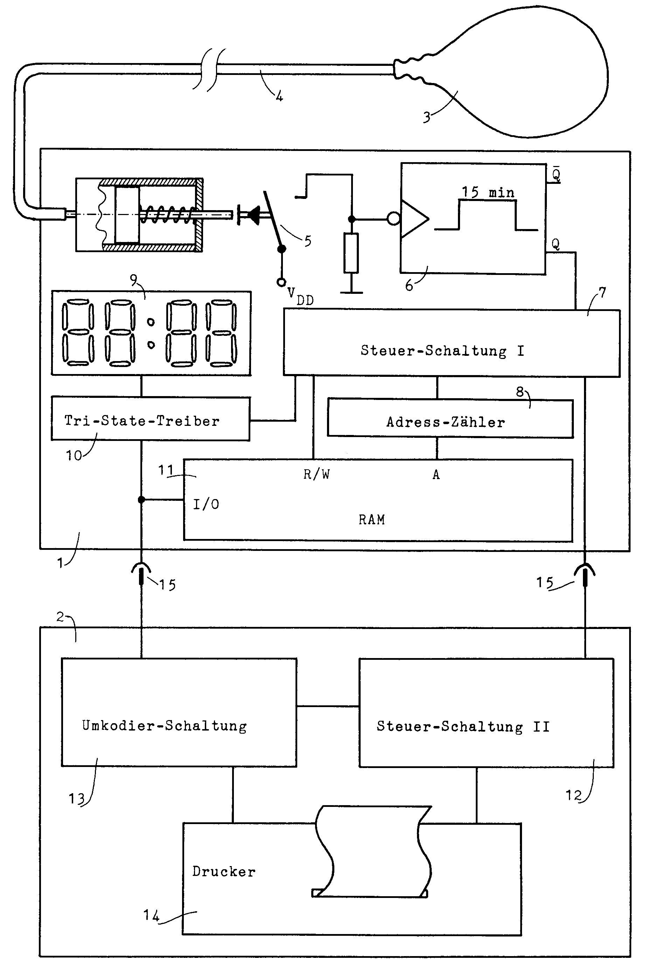

Ein Ausführungsbeispiel der Erfindung ist in der Zeichnung dargestellt und wird im folgenden näher beschrieben.

In der Registrier-Phase wird durch manuelles Zusammendrücken des Gummiballons (3) der dabei entstehende Luftdruck über einen dünnen Gummischlauch (4) auf einen kleinen Kolben geleitet, sodaß dieser eine Feder zusammendrückt und einen elektrischen Tastschalter (5) betätigt. Die dazu notwendige manuelle Druckkraft wird durch das Gummiballon-Material und durch die Federkonstante bestimmt.

Das Betätigen des Tastschalters (5) dient als Eingangssignal für das Monoflop (6), das daraufhin einen Impuls mit der Zeitdauer von 15 Minuten abgibt. Jede positive Monoflop-Impulsflanke bewirkt das Speichern der kodierten aktuellen Uhrzeit. Durch die festgelegte Monoflop-Impulsdauer werden Wachzustands-Zeitintervalle von 15 Minuten Dauer registriert.

Die Steuer-Schaltung I (7) gibt die notwendigen Steuersignale für jeden einzelnen Speichervorgang ab. Zuerst wird der Adress-Zähler (8) erhöht, sodaß eine neue Adresse am RAM-Adresseingang anliegt. Dann wird der Tri-State-Treiber (10) durchgeschaltet, damit die kodierte aktuelle Uhrzeit vom Digitaluhr-Modul (9) am RAM-Dateneingang anliegt, die dann durch ein Signal am RAM-Schreibeingang im RAM (11) gespeichert wird.

In der Ausdruck-Phase wird das Registriergerät (1) mit dem Ausdruckgerät (2) durch eine elektrische Steckverbindung (15) verbunden.

Die Ablaufsteuerung für den Ausdruckvorgang übernimmt die Steuer-Schaltung II (12), die in diesem Fall zusammen mit der Steuer-Schaltung I (7) arbeitet. Der Ausdruckvorgang beginnt damit, daß der Tri-State-Treiber (10) in den hochohmigen Zustand geschaltet wird, weil das RAM (11) die gespeicherten Daten auf die gemeinsamen Ein- und Ausgangsleitungen anlegen soll. Dann wird der Adress-Zähler (8) rückgesetzt und das RAM (11) über den RAM-Leseeingang in den Lese-Zustand geschaltet.

Die Umkodier-Schaltung (13) wandelt die im RAM (11) gespeicherten Daten in Druckersignale um, sodaß der Inhalt einer RAM-Speicherzelle als ein grafischer Balken von 15 Minuten Dauer mit entsprechendem Zeitbeginn in einem Zeitdiagramm durch den Drucker (14) ausgedruckt wird.

Ist der Druckvorgang für einen Wachzustands-Zeitintervall-Balken abgeschlossen, so erhöht die Steuer-Schaltung II (12) den Adress-Zähler (8), damit die nächsten im RAM (11) gespeicherten Daten umkodiert und ausgedruckt werden können.

Das gesamte ausgedruckte Wachzustands-Zeitintervall-Diagramm eines Patienten, das sich gewöhnlich über mehrere Schlafbereitschafts-Perioden erstreckt, dient dem Arzt zur Erstellung der Schlafstörungs-Diagnose.

## Patentansprüche

1. Medizintechnisches Gerät zur Diagnose und Therapie von Schlafstörungen,
gekennzeichnet durch einen Druckschalter (3,5), einen Monoflop (6), eine Steuer-Schaltung (7), einen Digitaluhr-Modul (9), einen elektronischen Schreib-Lesespeicher RAM (11), eine Umkodier-Schaltung (13) und einen Drucker (14) so arrangiert,
daß in einer Registrier-Phase einer Schlafstörung durch manuelles Zusammendrücken des Druckschalters mit bestimmter Druckkraft mit Hilfe des Monoflops (6) und der Steuer-Schaltung (7) ein binär kodiertes Wachzustands-Zeitintervall bestimmter Dauer, dessen Zeitbeginn von dem Digitaluhr-Modul (9) abgeleitet wird, in dem elektronischen Schreib-Lesespeicher RAM (11) gespeichert wird, und in einer späteren Ausdruck-Phase zur Erstellung der Schlafstörungs-Diagnose der gesamte Speicherinhalt mit Hilfe der Umkodier-Schaltung (13) und dem nachgeschalteten Drucker (14) in ein zeitliches Diagramm, das die Wachzustands-Zeitintervalle mit den jeweiligen Zeitbeginnpunkten dokumentiert, umkodiert und ausgedruckt wird.

2. Medizintechnisches Gerät nach Anspruch 1,
dadurch gekennzeichnet,
daß der Druckschalter aus einem Luftdruck-Fernauslöser besteht, sodaß durch manuelles Zusammendrücken eines kleinen Gummiballons (3) über einen dünnen Gummischlauch (4) pneumatisch ein am Auslöserende angebrachter elektrischer Tastschalter (5) betätigt wird.

3. Medizintechnisches Gerät nach Anspruch 1,
dadurch gekennzeichnet,
daß die Zeitkonstante des Monoflops (6) so eingestellt wird, daß sich ein Wachzustands-Zeitintervall von 15 Minuten Dauer ergibt.

4. Medizintechnisches Gerät nach Anspruch 1,
dadurch gekennzeichnet,
daß es aus zwei durch eine elektrische Steckverbindung (15) koppelbaren Teilgeräten (1) und (2) besteht, wobei das Registriergerät (1) die Wachzustands-Zeitintervalle speichert, und das Ausdruckgerät (2) die gespeicherten Daten des Registriergeräts (1) umkodiert und ausdruckt.

## Claims

1. Medical device for diagnostics and treatment of sleep disorders,
characterized by a pressure switch (3,5), a monoflop (6), a control circuit (7), a digital real-time clock module (9), an electronic write-read memory RAM (11), a recoding circuit (13) and a printer (14), arranged in such a way
that, in the phase during which a sleep disorder is recorded, manual compression of the pressure switch under a specific pressure force with the aid of the monoflop (6) and the control circuit (7), causes a binary coded waking state time interval of specific duration, the beginning of which is derived from the digital real-time clock module (9), to be stored in the electronic write-read memory RAM (11), and, in a subsequent printout phase for preparation of the sleep disorder diagnosis, the entire memory content is recoded and printed out with the aid of the recoding circuit (13) and the subsequent printer (14) in a time chart which documents the waking state time intervals with the corresponding starting time points.

2. Medical device pursuant to Claim 1,
characterized in that
the pressure switch consists of a remote air pressure actuator, so that an electrical sensor switch (5) fitted at the end of the actuator is operated pneumatically by manual compression of a small rubber ball (3), via a thin rubber hose (4).

3. Medical device pursuant to Claim 1,
characterized in that
the time constant of the monoflop (6) is set in such a way that a waking state time interval lasting for 15 minutes is obtained.

4. Medical device pursuant to Claim 1,
characterized in that
it consists of two partial devices (1) and (2), which can be coupled together by an electrical plug connection (15), the waking state time intervals being memorized by the recording device (1), while the printout unit (2) recodes and prints out the data stored in the recording device (1).

## Revendications

1. Dispositif médical destiné au diagnostic et à la thérapie de l'insomnie,
se caractérisant par un interrupteur à poussoir (3,5), un monoflop (6), une commutation de commande (7), un module à horloge numérique (9), une mémoire électronique de lecture et d'écriture RAM (11), une commutation de codage (13) et une imprimante (14), et conçu de telle sorte que
dans la phase d'enregistrement d'une insomnie par la compression manuelle de l'interrupteur à poussoir avec une force déterminée à l'aide du monoflop (6) et de la commutation de commande (7), un intervalle de temps d'éveil à codage binaire, d'une durée déterminée et dont le point de départ est fixé par le module à horloge numérique (9), soit enregistré dans la mémoire électronique de lecture et d'écritre RAM (11) pour être ensuite décodé puis imprimé ultérieurement afin d'établir le diagnostic d'insomnie de la totalité du contenu de la mémoire à l'aide de la commutation de codage (13) et de l'imprimante (14) et réaliser ainsi un diagramme de temps documentant les intervalles de temps d'éveil avec leur point de départ correspondant.

2. Dispositif médical selon la spécification 1,
se caractérisant par le fait
que l'interrupteur à poussoir se présente sous la forme d'une télécommande pneumatique, de manière à ce que la compression manuelle d'une petite poire en caoutchouc (3) déclenche pneumatiquement, par l'intermédiaire d'un mince tuyau en caoutchouc (4), un contacteur électrique (5) placé à l'extrémité du déclencheur.

3. Dispositif médical selon la spécification 1,
se caractérisant par le fait
la constante de temps du monoflop (6) soit réglée de manière à obtenir un intervalle de temps d'éveil d'une durée de 15 minutes.

4. Dispositif médical selon la spécification 1,
se caractérisant par le fait
qu'il se compose de deux dispositifs parties (1) et (2) pouvant être reliés par un raccord électrique enfichable (15), l'appareil d'enregistrement (1) mémorisant les intervalles de temps d'eveil et l'imprimante (2) décodant et imprimant les données mises en mémoire par l'appareil d'enregistrement (1).
